(19) Europäisches Patentamt
European Patent Office
Office européen des brevets

(11) **EP 3 618 815 B1**

(12) **EUROPEAN PATENT SPECIFICATION**

(45) Date of publication and mention
of the grant of the patent:
**06.12.2023 Bulletin 2023/49**

(21) Application number: **18795236.1**

(22) Date of filing: **03.05.2018**

(51) International Patent Classification (IPC):
*A61K 9/51* (2006.01)    *A61K 9/50* (2006.01)
*A61K 49/18* (2006.01)    *A61N 1/372* (2006.01)
*A61N 5/06* (2006.01)    *B82B 1/00* (2006.01)
*B82B 3/00* (2006.01)    *H05B 6/36* (2006.01)

(52) Cooperative Patent Classification (CPC):
**A61K 9/0024; A61M 37/00; A61N 1/372;**
A61M 2037/0007; A61M 2205/0266

(86) International application number:
**PCT/US2018/030942**

(87) International publication number:
**WO 2018/204687 (08.11.2018 Gazette 2018/45)**

(54) **PROPULSION AND CONTROL OF A MICRO-DEVICE**

ANTRIEB UND STEUERUNG EINER MIKROVORRICHTUNG

PROPULSION ET COMMANDE D'UN MICRODISPOSITIF

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.05.2017 US 201762501156 P**

(43) Date of publication of application:
**11.03.2020 Bulletin 2020/11**

(73) Proprietors:
• **Bionaut Labs Ltd.**
  **4672514 Herzliya (IL)**
• **Shpigelmacher, Michael**
  **Los Angeles, California 90064 (US)**

(72) Inventors:
• **SHPIGELMACHER, Michael**
  **Los Angeles, California 90064 (US)**
• **KISELYOV, Alex**
  **San Diego, California 92130 (US)**

(74) Representative: **Pearl Cohen Zedek Latzer Baratz
UK LLP**
**The Gridiron Building**
**One Pancras Square**
**London N1C 4AG (GB)**

(56) References cited:
WO-A1-2009/145405    US-A1- 2007 225 634
US-A1- 2008 255 543    US-A1- 2009 299 335
US-A1- 2011 200 434    US-A1- 2013 129 824
US-A1- 2013 263 599    US-A1- 2015 013 304
US-A1- 2016 136 104    US-B1- 6 240 312
US-B2- 9 067 047

**Description**

## BACKGROUND OF THE INVENTION

A number of techniques have been proposed for magnetically-actuated propulsion of microscopic objects (sometimes referred to as micro-robots). For example, U.S. Patent 8,768,501, describes methods and systems for the fabrication and application of magnetically actuated propellers (MAPs), with typical feature size in the range of 20 nanometers up to 100 microns (micrometers), in one spatial dimension.

Another technique for magnetic actuation is the selective and directional actuation of elastomer films, utilizing magnetic anisotropy introduced by chains of $Fe_3O_4$ magnetic nanoparticles (MNPs). *See* Mishra et al., "Selective and directional actuation of elastomer films using chained magnetic nanoparticles," Nanoscale 8 (2016), pages 1309-1313. Under uniform magnetic fields, or field gradients, dipolar interactions between the MNPs favor magnetization along the chain direction and cause selective lifting.

US 2008/0255543 discloses a body-insertable apparatus for introduction into a subject, to inject medical agent stored in a casing into a desired part in the subject. The body-insertable apparatus includes a fixing unit which fixes the casing to the desired part, and a projecting unit which projects an injection needle from the casing for injecting the medical agent. The fixing unit and the projecting unit are driven by a driving source.

US 2011/0200434 discloses a microparticle including an oblong flexible tail able to propel the microparticle in a solution along a trajectory using beats transverse to the trajectory. The tail includes at least one magnetic element such that the magnetic element causes beats of the tail under the action of an external alternating magnetic field non-collinear with the trajectory. The microparticle further includes a head mechanically connected to a proximal end of the tail. The microparticle includes at least one layer of material formed from one piece and including the tail and the head, the dimensions and/or shape of the head being selected such that the beats of the proximal end of the tail are limited with respect to the beats of the distal end of the tail and such that the head does not perform a complete revolution around an axis parallel to the trajectory under the effect of the external alternating magnetic field.

US 2007/0225634 discloses lumen-traveling devices capable of traveling within a body lumen and of controlled material release. The lumen-traveling devices may include a propelling mechanism to produce movement thereof within the lumen, as well as additional components such as a sensor, a material release portion, and/or control circuitry.

Accordingly, there is a need for devices able to move in a viscoelastic media, by at least one applied stimulus field (SF).

## SUMMARY OF THE INVENTION

In one embodiment, this invention provides a device for implanting in a biological tissue and adapted to move in a viscoelastic media, the device comprising:

- a main-body comprising a first material (**M1**) and having a direction in the viscoelastic media, and being configured to change its direction in the viscoelastic media upon application thereto of a first stimulus field (**SF1**) at a first threshold (**T1**); and

- one or more memory shaped elements (MSE) externally attached to the main-body, each of the MSEs having a first configuration and comprising a second material (**M2**) being a stimulus-responsive shape-memory material, said second material comprises an elastomer, and wherein the MSE is configured to adopt a second configuration upon application thereto of a second stimulus field (**SF2**) at a second threshold (**T2**), thereby propelling the main-body through the viscoelastic media.

In one embodiment, the second material (**M2**) is different from the first material (**M2**≠**M1**). In one embodiment, **SF1** and **SF2** are of the same nature (i.e., based on the same physical principle, for example, both fields are ultrasound (US) fields, magnetic fields, electric fields or electromagnetic fields) and the same direction; and wherein **T2** is larger than **T1**. In one embodiment, the material of at least some of the MSEs are different one from another ($M2_i \neq M2_j$, $i \neq j$). In one embodiment, at least one of **M1** and **M2** comprises a form of micro- or nano- particles. In one embodiment, the first or second configuration of the MSE is selected from a group consisting of: an elongated shape, a film, a wire, a string, a strip, a plug, a sheet, a membrane, flagellum, coil, helix, arm, joint and any combination thereof. In one embodiment, the application of the **SF2** comprises cycles of the second stimulus field above and below the second threshold (**T2**).

In one embodiment, the main-body further comprises at least two fins, configured to steer the direction of the main-body. In one embodiment, the fins comprise the first material (**M1**). In one embodiment, the fins comprise a polarity direction at an angle relative to the main-body. In one embodiment, the fins are externally and symmetrically attached to the main-body. In one embodiment, the fins are configured to tilt relative to the main-body.

In one embodiment, the main-body further comprises a sealable cavity and when the MSE is in the first configuration the cavity is closed and in the second configuration the cavity is open. In one embodiment, the sealable cavity is configured to temporarily accommodate at least one of: a therapeutic entity, a therapeutic load, a diagnostic load, or a combination thereof. In one embodiment, the sealable cavity is configured to temporarily accommodate an explosion material, configured to propel the main-body.

In one embodiment, the device further comprising a sensitive sealing lid, configured to temporarily seal the cavity; wherein the sensitive sealing lid is configured to be opened responsive to an environmental threshold. In one embodiment, the MSE is configured as a sealing lid for the cavity; and wherein the configuration of the MSE opens and/or closes the sealable cavity. In one embodiment, the MSE comprises a first arm and pulls and/or pushes a sealing-lid of the cavity upon application of **SF2**. In one embodiment, the first arm comprises at least one element selected from: a spring, a helical spring, a leaf spring, a rod, a shaft, a pole and a bar. In one embodiment, the main-body further comprises a cavity and wherein the MSE comprises a second arm, configured to push a substance accommodated within the cavity out of the cavity upon application of **SF2**.

In one embodiment, this invention provides a system comprising:

- a device described herein; and
- a remote controlling module configured to control the application of **SF1** and **SF2**.

In one embodiment, the remote controlling module comprises at least one inducer for a stimulus field selected from: magnetic, electric, acoustic, ultrasound, heat, X-ray, radio-wave and any combination thereof.

In one embodiment, the system further comprises a delivery and/or retraction module, configured to deliver and/or retract the device to and/or from a specific location selected from: *in vitro*, *ex vivo*, *in vivo* in a mammalian subject, and *in vivo* in a human patient. In one embodiment, the delivery and/or retraction module comprises an attachment element selected from: a magnetizable needle, expandable magnetic element, magnetizable surface, pneumatic element, electromagnetic element, ultrasonic element, deployable mesh, deployable micro-net, suction element, and any combination thereof. In one embodiment, the remote controlling module comprises a monitoring-device, configured to locate and display location and orientation of the device within the viscoelastic media.

In one embodiment, this invention provides a method comprising applying at least one of the stimulus fields (SF) to a device described herein to manipulate motion of the main-body within the viscoelastic fluid of a subject. In one embodiment, manipulation comprises: steering the main-body to a desired direction via an **SF1** corresponding to the lower threshold (**T1**); and/or propelling the main-body by modifying the configuration of the MSE, via an **SF2** corresponding to the second threshold (**T2**).

In one embodiment, the method further comprising at least one of:

- externally loading the device's cavity with a selected load;
- delivering the device into a treated subject;
- monitoring the device's location and orientation within the viscoelastic media;
- releasing the selected load from the cavity at a desired location;
- imaging the subject to locate the device for further diagnostic information; or
- retracting the device from a pre-determined location.

In one embodiment, the step of delivering comprises at least one of: injecting, providing for swallow, penetrating via catheter. In one embodiment, the step of releasing the selected load comprises modifying the configuration of the MSE via the **SF2** at the second threshold (**T2**), such that the cavity's sealing lid is opened. In one embodiment, the step of releasing the selected load comprises opening the sensitive sealing lid, by providing a selected environmental threshold.

## BRIEF DESCRIPTION OF THE DRAWINGS

The subject matter regarded as the invention is particularly pointed out and distinctly claimed in the concluding portion of the specification. The invention, however, may best be understood by reference to the following detailed description when read with the accompanying drawings in which:

Figs. **1A**, **1B** and **1C** schematically demonstrate a device having a flagellum, according to some embodiments of the invention;

Figs. **2A** and **2B** schematically demonstrate a device having a cavity, according to some embodiments of the invention;

Figs. **3A** and **3B** schematically demonstrate another device having a cavity, according to some embodiments of the invention;

**Figs. 4A** and **4B** schematically demonstrate another device having a cavity, according to some embodiments of the invention;

**Figs. 5A** and **5B** schematically demonstrate another device having a cavity, according to some embodiments of the invention;

**Fig. 5C** schematically demonstrates another device having a cavity, according to some embodiments of the invention;

**Figs. 6A, 6B, 6C, 6D, 6E** and **6F** schematically demonstrate a device with fins, according to some embodiments of the invention; and

**Fig. 7** schematically demonstrates a system, according to some embodiments of the invention.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

## DETAILED DESCRIPTION OF THE INVENTION

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

The term "*device*" herein denotes any object that is implantable in biological tissue. The terms "*carrier device*" and "*carrier*" herein denote a device that is capable of carrying and releasing a medical payload into the tissue. The term "*medical payload*", or equivalently the terms "*payload*" and "*cargo*" used in a medical context is understood herein to include any substance or material, a combination of several relevant therapeutic materials, diagnostics or a combination of therapeutic and diagnostics. In certain embodiments of the invention, a fluid payload is used; the term "*fluid*" herein denotes that the payload is capable of flowing. In certain embodiments of the present invention, a solid payload is used; the term "*solid*" herein denotes that the payload can be released in the form of discrete particles. A device may be fabricated by known manufacturing techniques, including, but not limited to, 3D printing, molding, casting, etching, lithography, thin-film technologies, deposition technologies, and the like.

In various embodiments of the present invention, carrier devices are miniaturized for implantation in biological tissues. The term "*miniaturized*" (with reference to a device) herein denotes a device of small size, including, but not limited to: devices of millimeter to centimeter scale; devices of micrometer ("micron") scale, referred to as "micro-devices"; devices of nanometer scale (including hundreds of nanometers), referred to as "nano-devices." Not only are the devices themselves of the size scales as indicated above, but the devices' individual components are also of comparable scale.

According to some embodiments of the invention, a micro-/nano- device is provided comprising elastomer films with chained magnetic particles, which are configured for selective and directional actuation, for applications such as: propulsion, steering, and controlling the motion of the device. According to some embodiments, the elastomer films can control elements of the device such as open and/or close compartments thereof.

According to some embodiments, the diameter or actual length of the overall device is selected from: between 100 and 5,000 micrometers, between 10 and 100 micrometers, between 1 and 10 micrometers, between 200 and 1,000 nanometers, and any combination thereof. According to some embodiments, the diameter or actual length of the overall device is from 200 nanometers up to 5,000 micrometers.

A skilled artisan will appreciate that, memory shaped elements (MSEs), may refer according to some embodiments, to smart materials that are able to return from a deformed state (deformed under an applied stimulus field) to their original shape, when the stimulus field is removed or at least under (or alternatively above) a predefined threshold/s.

A skilled artisan will appreciate that, the phrase "applying the stimulus field corresponding to a threshold" or similar phrases may refer to applying the stimulus field such that it crosses a threshold (above or below, depending on the specific application), such that at least one material of the device reacts. For a non-limiting example, a thermal stimulus field can be applied where: for a heating stimulus a reaction occurs above a predetermined temperature (such as material melting) and for a cooling stimulus a reaction occurs below a predetermined temperature (such as material freezing).

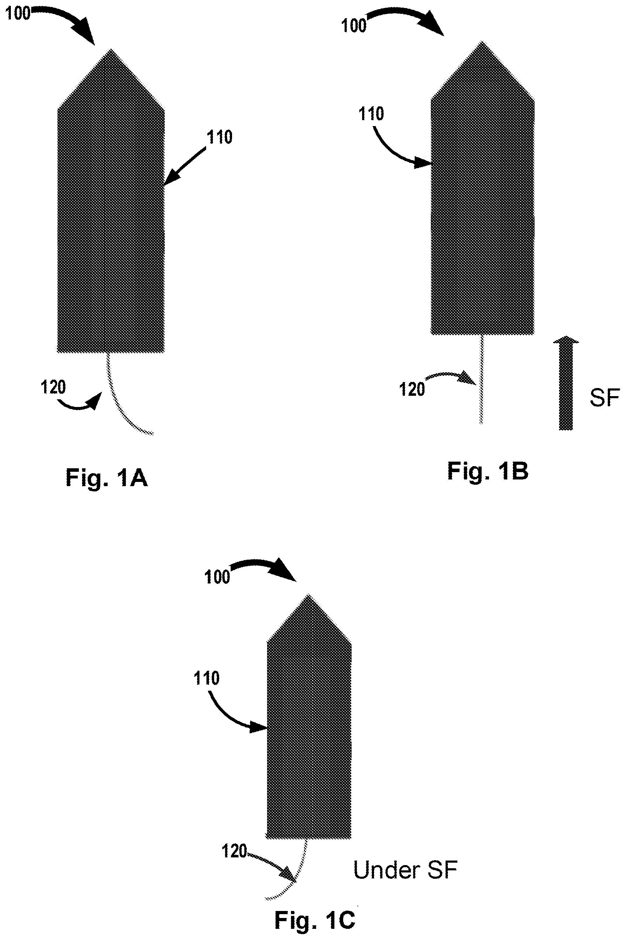

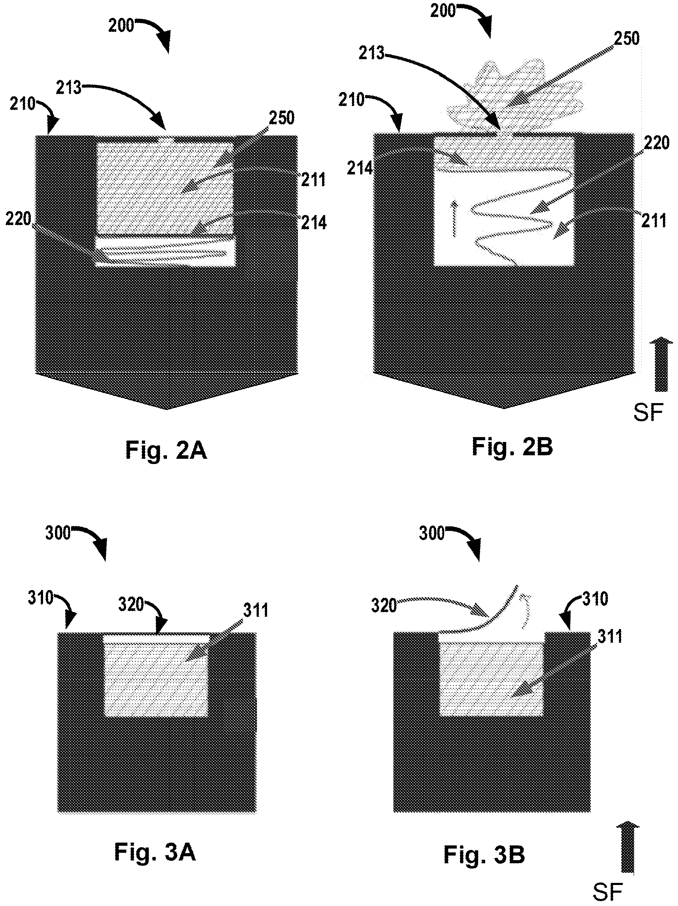

Reference is now made to **Figs. 1A-1C** and **2A-2B**. According to some embodiments, a device [100, 200] is provided and configured to move and travel in a viscoelastic media, responsive to an application of at least one stimulus field (SF); the device [100, 200] comprising:

- a main-body [110, 210] comprising a first material (M1), M1 is configured to respond to an applied SF corresponding to (higher- or lower- than) a first threshold (T1); and
- one or more memory shaped elements (MSEs) [120, 220] comprising a second material (M2), M2 is configured to deform responsive to an applied SF corresponding to (higher- or lower- than) a second threshold (T2);

wherein M1 is selected to enable manipulation of the main-body's direction in the viscoelastic media; and wherein M2 is selected to enable manipulation of MSE shape.

According to some embodiments, the MSE is configured to return to its original shape, once the SF is removed, or applied respectively (to the above mentioned) lower- or higher- than the second threshold. According to some embodiments, the SF is applied in a pulsatile (on/off) fashion.

According to some embodiments, the shape/s of the MSE is/are configured to propel the main-body in the viscoelastic media.

According to some embodiments, the second material is different from the first material (M2≠M1). According to some related embodiments, the materials M1 and M2 are both configured to react (respond/deform, respectively) to the same type of same SF. According to some related embodiments, the materials M1 and M2 are selected, such that upon the application of the SF, their corresponding first- and second- thresholds (T1≠T2) initially enable the activation of the first material (SF causing the main-body to respond) and then, with a higher SF application enable the activation of the second material (SF causing the MSE to deform); or vise-versa: initially activate the second material and then with a higher application of the SF activate the first material; depending on the selected application. Examples with the application of magnetic stimuli field are described in Examples 1 and 2.

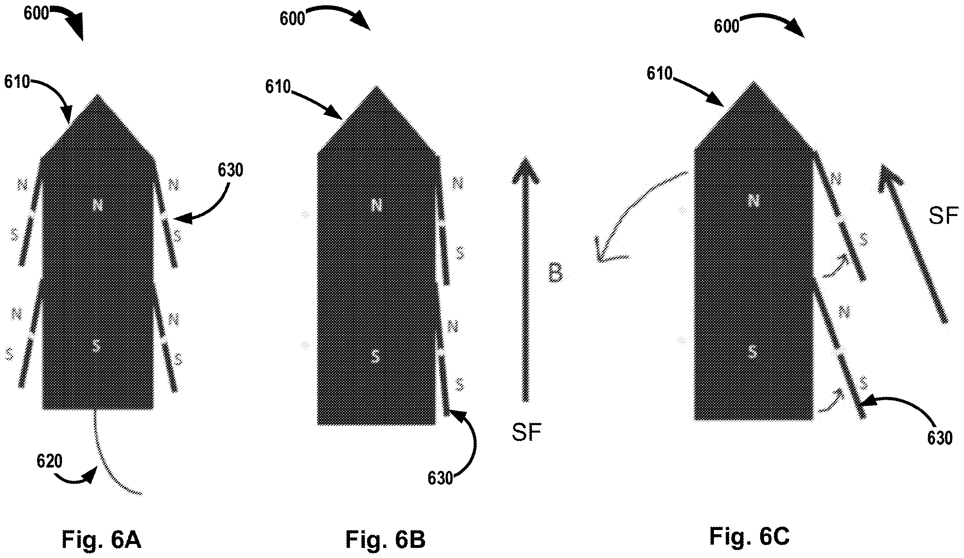

According to some embodiments, the second material (M2) is selected such that the applied SF (corresponding to the second threshold T2) is configured to deform the MSE and align its shape along the direction of the applied SF. **Fig. 1A** demonstrates an MSE [120] in its original shape, before application of the SF; and **Fig. 1B,** demonstrates the aligned MSE [120], during the application of the SF corresponding to the second threshold (**T2**). In **Figs. 1A-1C** and also in **Figs. 6A-6E** the MSEs [120,620] are designed as flagellum/flagella configured to propel the main-body in the viscoelastic media.

According to some embodiments, the second material (M2) is selected such that the applied SF (corresponding to the second threshold T2) is configured to deform the MSE into a predetermined shape (different from its original shape). **Fig. 1A** demonstrates the MSE [120] in its original shape (twisted to the right side), before the application the SF; and **Fig. 1C,** demonstrates the predetermined deformed shape MSE [120] (twisted to the left side), during the application of SF corresponding to a second threshold (**T2**). Another example is in **Fig. 2A** which demonstrates the MSE [220] in its original (compressed) shape, before the application the SF; and where **Fig. 2B,** demonstrates the predetermined deformed (expanded) shape MSE [220], during the application of SF corresponding to a second threshold (**T2**).

According to some embodiments, in the case of a plurality of MSEs, their materials M2 can be selected to be different, at least for some of the MSEs, or different per each MSE; namely selecting materials ($M2_1$, $M2_2$, ... $M2_n$), such that each of the MSEs deforms under an applied SF corresponding to its respective second threshold ($T2_1$, $T2_2$, ... $T2_n$).

According to some embodiments, the main-body comprises a shape selected from elongated, axisymmetric, centrosymmetric, chiral, random and any combination thereof.

According to some embodiments, the response of the main-body and/or sections thereof to the SF comprises at least one of: rotate, modify orientation, propel, oscillate, undulate, translate, expand, constrict, tilt away, tilt towards and a combination thereof.

According to some embodiments, the viscoelastic media comprises a material selected from: human blood, mammalian blood, biological tissue, biological organ and/or system, natural gel, synthetic gel, lymph, bile and a combination thereof.

According to some embodiments, the stimuli field is selected from: magnetic, electric, electro-magnetic, optical, acoustic, ultrasound, photoacoustic, radio waves, thermal, pH, solution, immunological, redox, thermal, enzymatic, protein, X-ray, cellular compartment-specific environment, and a combination thereof.

According to some embodiments, at least one of the stimuli fields is externally applied. According to some embodiments, at least one of the stimuli fields is internally applied. According to some related embodiments, the internally applied stimuli field is location related or dependent, namely depends upon the device's current location; for a non-limiting example, a pH level at a specific organ within a human (or other mammalian) body.

According to some embodiments, at least one of the first- and second- materials comprises a form of micro- or nano-particles.

According to some embodiments, at least one MSE comprises an elastomer material (as mentioned in the background) having a configuration selected from a group of: an elongated shape, a film, a wire, a string, a strip, a sheet, a plug, a membrane, flagellum, coil, helix, arm, joint and any combination thereof. Embodiments disclosed herein for an elastomer film also apply to other configurations from the list presented herein above.

According to some embodiments, at least one MSE comprises a material selected from: composite memory polymer that contains embedded electric, magnetic-sensitive material, acoustic-sensitive material, microwires, diverse microparticles, microirregularities, layered 2D/3D nano-/microstructures, pH-sensitive material, redox-sensitive material, specific enzyme-sensitive coating that triggers reversible or irreversible topological change, and any combination thereof.

According to some embodiments, and as demonstrated at least in **Figs. 1A-1C,** at least one MSE [110] is externally attached to the main-body (for example a flagellum [120]), configured to propel the main-body in the viscoelastic media, responsive to the application of the SF corresponding to the second threshold (**T2**). According to some related embodiments, the SF application comprises cycles of the SF above- and below- the second threshold (**T2**). According to some related embodiments, the cycles of application can be a frequency application of the stimuli field.

According to some embodiments, and as demonstrated in **Figs. 6A-6C,** the main-body [600] further comprises at least one fin [630], configured to steer the direction of the main-body. According to some embodiments, the fins are configured to tilt relative to the main-body [610], thereby rotate, propel and/or turn the main-body within the viscoelastic media, as demonstrated in **Figs. 6A-6C:** before the application of the SF (as in Fig. 6A), and during the application of the SF corresponding to a first threshold (T1), as in **Figs. 6B** and **6C** for different directions of the SF.

According to some embodiments, the fins are smaller than the main-body. According to some embodiments, the fins are positioned in an axisymmetric arrangement. According to some embodiments, at least one of the fins is flexible. According to some embodiments, at least one of the fins is rigid. According to some embodiments, the fins are attached to the main body by pins and/or joints. According to some embodiments, the fins are attached to the main body via adhesive elements or methods.

According to some embodiments, the fins [630] comprise a third material (M3). According to some embodiments, materials M1 and M3 both configured to react to the same SF. According to some embodiments, the fins comprise the first material (M3=M1). According to some embodiments, the fins have the same fixed polarity direction as the main-body. For example, and as demonstrated in **Figs. 6A-6F,** the direction of magnetization polarity (or alternative force field vector) is parallel or slightly tilted relative to the axis of symmetry of the main-body [610].

According to some embodiments, and as demonstrated for devices [200, 300, 400, 500, 800] in **Figs. 2A-2B**, **3A-3B**, **4A-4B**, **5A-5B** and **5C** the main-body [210, 310, 410, 510, 560] further comprises a sealable cavity [211,311,411,511,561]. According to some embodiments, the volume of the cavity is selected from between 5% and 95% of the main-body. According to some embodiments, the volume of the cavity is selected from 5%, 10%, 15%, 20%, 25%, 30%, 35%, 40%, 45%, 50%, 55%, 60%, 65%, 70%, 75%, 80%, 85%, 90% or 95% of the volume of the main-body.

According to some related embodiments, the sealable cavity is configured to temporarily accommodate a predetermined load selected from at least one of: jet load, diagnostic load, therapeutic load, therapeutic entity and a combination thereof. According to some embodiments, the sealable cavity is configured to temporarily accommodate multiple therapeutic entities and multiple diagnostic loads in predetermined combination thereof.

According to some embodiments, the jet material is remotely activated and the jet's torque is configured to propel the main-body in the viscoelastic material. According to some embodiments, the application of the SF at a predetermined level activates the jet propulsion-generating material.

According to some related embodiments, the MSE [320, 420, 520, 562] is configured to control the opening and closing of the cavity, responsive to the application of the SF corresponding (above- or below-) to the second threshold (T2); for example, by opening a sealing lid [320,412,512,562] of the cavity, as respectively shown in **Figs. 3B, 4B, 5B** and **5C.**

According to some related embodiments, the MSE [220,320,420,520,562] is configured to release a selected load accommodated within the cavity [211, 311, 411, 511, 561], responsive to the application of the SF corresponding (above- or below-) to the second threshold (T2). For example, by pushing or extruding the load via a small opening hole of the cavity [213], as demonstrate in **Fig. 2B,** or by opening the cavity as demonstrated in **Figs. 3B, 4B, 5B** and **5C.**

According to some related embodiments, therapeutic entities can be loaded into the cavity, and comprise at least one of: radionuclides, alpha-particles and neutron emitters, small peptides, peptoids, antibodies, antibody-drug conjugates, modified antibodies and their derivatives as exemplified but not limited to light chain antibody constructs, nucleic acids as exemplified but not limited to aptamers, antisense oligonucleotides, RNAi, siRNAs, shRNAs, miRNAs.

In some embodiments, the therapeutic load can comprise components of CRISPR-Cas9 or related gene editing molecules. In some embodiments, the therapeutic load can include vaccines as exemplified but not limited to the Bacillus Calmette-Guerin vaccine. In some embodiments, the therapeutic load can include oncolytic viruses as exemplified but not limited to Talimogene laherparepvec (OncoVEX GM-CSF). In some embodiments, the therapeutic load can include specialized cells and or cell therapy as exemplified by but not limited to CART cells or pluripotent stem cells. In some embodiments, the load can include diagnostics and contrasting agents including but not limited to radio-, MRI- or ultrasound contrast agents. In some embodiments, the cavity described therein can contain active agents as solids, solutions or alternative formulations including gels, sols, suspensions, nano- or microformulations of therapeutic agents including but not limited to micelles, liposomes, mesoporous silica-, carbon nanotube-mediated carriers their composites or alternative particles that supply intended therapeutic load of an agent or their mixtures and fit the cavity.

According to some embodiments, and as can be seen from **Figs. 2A-2B** and **3A-3B** (but not limited to), the sealable cavity [211,311] is configured to temporarily accommodate an expulsion material, configured to propel the main-body [210,310]. According to some embodiments, the expulsion material is configured to be triggered by a predetermined threshold to the applied SF.

According to some embodiments, the device further comprises a sensitive sealing lid, configured to temporarily seal the cavity. The sensitive sealing lid is configured to be opened (for example dissolve, melt, bend) responsive to a threshold to an environmental local field (not by the applied SF) selected from: acoustic, ultrasound, temperature, pH, redox, enzymatic, protein, cellular compartment.

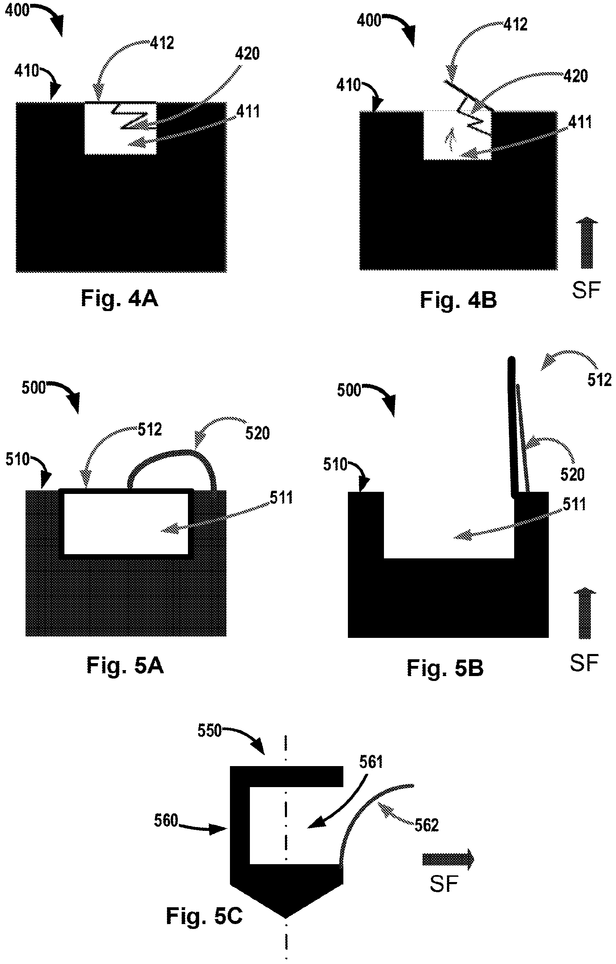

According to some embodiments, and as demonstrated in **Figs. 3A-3B** and **5C,** the MSE is configured as a sealing lid [320,562] for the cavity [311,561]; and wherein manipulation of the MSE's shape is configured to open and/or close the cavity.

According to some embodiments, and as illustrated in **Figs. 4A-4B** and **5A-5B,** the MSE is configured as a first arm [420,520], configured to pull and/or push a sealing-lid [412,512] of the cavity. **Figs. 4A-4B** illustrate the first arm [420], configured to open/close the sealing lid [412] from within the cavity [411], while **Figs. 5A-5B** illustrate the first arm [520], configured to open/close the sealing lid [512] from an external side of the cavity [511].

According to some embodiments, and as demonstrated in **Figs. 2A-2B,** the MSE is configured as a second arm [220], configured to push a tray [214] on which the load is accommodated, and thereby to push that load out of the cavity [211], responsive to the application of the SF corresponding to a second threshold (T2).

According to some embodiments, at least one of the first- and second- arms is selected from: a spring, a helical spring, a leaf spring, a rod, a shaft, a pole, and a bar.

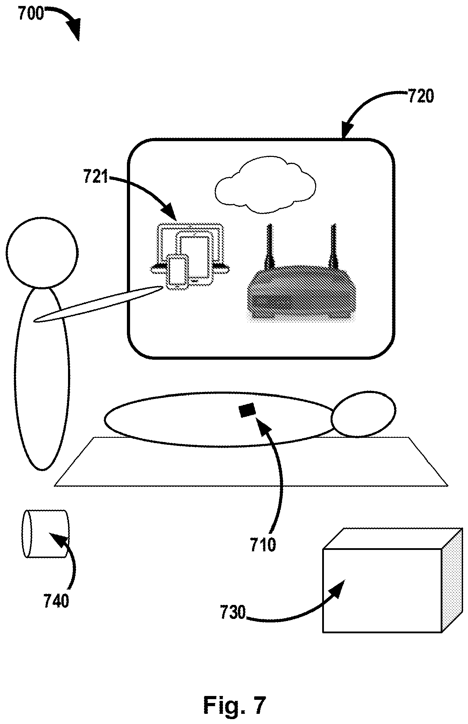

According to some embodiments of the invention, and as demonstrated in **Fig. 7,** a system [700] is provided comprising:

- At least one device [710] of one of the above-mentioned embodiments [100, 200, 300, 400, 500, 600, 550]; and
- a remote controlling module [720] to control the application of the stimuli fields (SF), thereby manipulating the direction of the main-body in the viscoelastic media and to the shape of the MSE.

According to some embodiments, materials of one device are different from another, accordingly their corresponding thresholds.

According to some embodiments, the remote controlling module [720] comprises a monitoring device [721], configured to locate and display the location and orientation of the device [710] within the viscoelastic media.

According to some embodiments, the remote controlling module [720] comprises an input device [721] to be handled by a caregiver, configured to provide instructions to the device's [710] motion within the viscoelastic media.

According to some embodiments, the remote controlling module [720] comprises at least one inducer [730] for a stimulus field selected from: magnetic, electric, piezoelectric, acoustic, ultrasound, heat, X-ray, radio-wave, optical and any combination thereof.

According to some embodiments, the magnetic field inducer [730] comprises a set of permanent magnets and/or conducting coils (such as Helmholtz or Maxwell coils) generating an arbitrary magnetic field vector at predefined location, where the main-body and MSE are located. Such magnetic field vector can be adjusted to control direction of the main body and shape of the MSE. According to some embodiments, a combination of coils and/or fixed magnets can generate the magnetic field.

According to some embodiments, the remote controlling module [720] is configured to control features of the SF selected from: power, intensity, frequency and direction; for a non-limiting example: to focus an ultrasound via a series of diverse transducers to adjust to a specific topology and depth. According to some embodiments, the remote controlling module [720] is configured to control a combination of aforementioned external stimuli to control both the main body and MSE in a synergistic or discrete fashion; for a non- limiting example, using electromagnetic and ultrasound stimuli to remotely control specific aspects of the device's [710] propulsion.

According to some embodiments, the system [700] further comprises a delivery and/or retraction module [740], configured to deliver and/or retract the device to- and/or from- a specific location selected from: *in vitro*, *ex vivo*, *in vivo* in a mammal, or *in vivo* in a human patient. According to some embodiments, the module comprises an attachment element selected from: magnetizable needle, pneumatic element, expendable magnetic element, magnetic surface, electromagnetic element, ultrasonic element, deployable mesh, deployable micro-net, suction element, and a combination thereof.

According to some embodiments, the delivery and retraction module is aimed at controlled delivery and collection of nano- or micro-devices to and from a specific location prior to and after actuation with external stimuli and cargo delivery. According to some embodiments, the module can comprise one or several structural elements to deliver and collect said nano- or micro-devices. According to some embodiments, the module can contain specific design to secure single or multiple insertions for *in vitro*, *in vivo* or patient applications. According to some embodiments, the module can contain a magnetic or magnetizable needle for injecting and collecting the nanos or micro-devices. According to some embodiments, the module can contain alternative delivery techniques based on electromagnetic, ultrasound or pneumatics-based devices. According to some embodiments, the module can contain alternative collection techniques as exemplified but not limited to deployable mesh, micro-net or suction. According to some embodiments, the magnetic needle can be designed to accommodate a standalone device or a device in a matrix to secure precise delivery. According to some embodiments, the magnetic or magnetizable needle can be kept in the injection matrix *in vitro*, *in vivo* or in patient for the duration of treatment or retracted and reintroduced for device collection.

**EXAMPLE 1.**

An example with a magnetic stimuli field is provided. In this example, a steering and propulsion device is provided to move or travel in a viscoelastic media on the nano-/micro-/milli- meter scale, using external magnetic fields. The materials of the device include a combination of elastomer-based flagellum for propulsion and a magnet-based main-body and fins for directional steering. Such a device can be used to propel a particle inside a human body via the tissue, carry medical payloads (therapeutics or diagnostics) or conduct minimally invasive surgery.

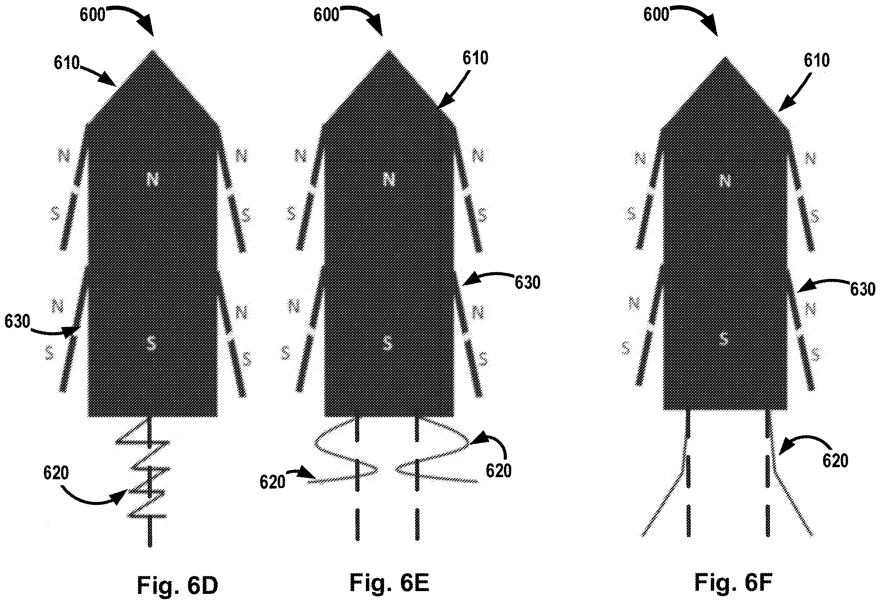

As shown in **Figs. 6D-6E,** particle (device [600]) comprises three main components:

- the main-body [610] of the magnetic particle, with a fixed polarity, corresponding to the desired direction of motion, based on an embedded magnetic component with a sufficiently strong magnetic moment;
- smaller magnetic fins [630] attached to the main-body symmetrically all around its axis (cylindrical symmetry); where each fin has a fixed polarity, aligned with the polarity of the main-body of the particle, based on an embedded magnetic component in the fin with a sufficiently strong magnetic moment. Such fins can be produced, for example, from the elastomer films that are described in Mishra *et al.*, or alternatively by other suitable techniques known in the art. Such films comprise for example $Fe_3O_4$ magnetic nanoparticles (MNPs) and thermoplastic polyurethane (TPU). The films are nanocomposites comprising the polymer and the magnetic nanoparticles. Assembly of the MNPs into chains causes a directional dependence in the magnetostatic energy, allowing for anisotropic actuation of the composite in 3D. The fins are attached to the main-body with a flexible attachment point and/or are made of flexible material, so they can tilt or "flap" when placed in an external magnetic field (since they are magnetic, they can tilt to align with the external magnetic field); in this case the main-body of the particle is also subject to a rotating torque aligning it with the external magnetic field; since the body is larger than the fins it can tilt more slowly, subject to drag in viscoelastic media, allowing the fins to "flap" relative to the body; and
- a flagellum (or multiple flagella as in Fig. 6E), at the tail end of the main-body, made of elastomer with embedded magnetic nanoparticles (MNPs).

In this example, the MNPs in the flagella are based on a magnetic material **M2,** which is different (in terms of magnetic permeability, magnetic moment) from the magnetic material used for the main-body and fins (M1, M1' respectively); the reason for such material selections are as follows.

Particle motion is controlled by an external magnetic field:

$$B = B1 + B2, \qquad \textit{Eq. 1}$$

where:

- B1 is a fixed low amplitude (low power) steering component (changing direction only when the particle is required to turn), and
- B2 is a varying amplitude, high power, on-off pulse component, which is in charge of propulsion;

both B1 and B2 vectors are in the same direction.

When B=B1 (meaning B2=0) the flagella remain in their relaxed position, since the flagella are based on elastomers with embedded magnetic material **M2,** with a magnetic moment that is too weak to generate sufficient torque for flagella movement under field B1.

In contrast, the materials **M1, M1'** used respectively for the particle body and fins have a magnetic moment large enough to generate rotational (steering) particle movement under field B1.

Importantly, material M2 does not necessarily have a lower magnetic moment compared to M1, M1' per unit volume or mass. However, M2's magnetic moment is too weak relative to the minimal threshold needed for flagella activation (i.e., field B1 generates torque strong enough to steer the main-body and fins, but not strong enough to activate the flagella). The minimal threshold (T2) to activate the flagella depends on elastomer mechanical characteristics, such as dynamic moduli, flagella geometry and size, as well as surrounding medium rheology. The minimal threshold (T1) to steer the main-body and fins depends on the surrounding medium rheology, as well as particle geometry and size.

In summary, the flagella do not change their shape under the weak magnetic field B1. Only when B is clearly greater than B1 (i.e., B2>>0) the external field is high enough to activate the flagella and make them change their shape.

The on-off changes in flagella shape as a result from the on-off pulses of B2 generate the motion of flagella that propels the particle forward.

**Figs. 6E** and **6F** demonstrate a configuration where the flagellum has two possible configurations of minimal potential (symmetrical to each other). In each of those configurations the flagellum is curved, either to one side or to the other. When a strong external magnetic field B is switched on, the flagellum straightens (marked with dashed lines), reaching a potential local minimum point (in the middle between the two symmetrical global potential minima points). This configuration is referred to as a bi-stable structure, supported by two orthogonal curvature axes (parallel to the two sides of the rectangular elastomer sheet). An example of such a structure is a "snap bracelet". When the external magnetic field is switched off, the flagellum snaps back to either one of the potential minima points (with equal probability). When the field B2 component is repeatedly switched on-off, this on average results in a flip-flop motion between the two potential minima configurations of the flagellum (analogously to a fish tail fin motion), thus propelling the particle (device [610]) forward. According to some embodiments, when field component B2 is kept switched off, the flagellum rests in one of the two stable potential minima configurations (not flip-flopping). Only when the B2 component is switched on, the flagellum arrives at the unstable middle position, from which it will randomly flip to one of the two stable positions, once the field component B2 is switched off.

**Fig. 6E** illustrates a configuration where there are two flagella, which have symmetrical curved shapes when there is no strong external magnetic field B2 (similar to a frog's legs). When B2 is large, the flagella straighten (marked with dashed lines), pushing the particle forward.

**Fig. 6D** shows a configuration where the flagellum in its relaxed position (without strong external field B2) has a folded accordion shape. When external field B2 is switched on, the flagellum straightens (marked with dashed line), pushing the particle forward.

According to some embodiments, each flagellum comprises an elastomer sheet with a particular shape (in three-dimension). To clarify, **Fig. 6A-6F** show cross-sections of the particles and their flagella, rendering each flagellum as a two-dimensional curve. Many other flagella configurations are possible, resulting in propulsion of the particle forward.

According to some embodiments, when B changes its direction the main-body and fins tilt to align with the direction of B, steering the particle in the desired direction; as shown in **Figs. 6A-6C** (before SF application (**6A**) and for two different SF directions (**6B** and **6C**)).

The combination between the steering component and the on-off propulsion pulse component is configured to generate a directed and accurate remotely-controlled motion of the device [600] in viscoelastic media.

According to some embodiments, the external magnetic field can be generated by permanent magnets, Helmholtz, Maxwell coils or a combination thereof around the target area (the current location of the device). The exact shape and size of fins, particles and flagella can be optimized to improve mobility in specific viscoelastic media.

The strength of the relevant magnetic fields B1, B2 can range anywhere between single-digit Gauss to single-digit Tesla (depending on particle size and geometry, materials used, and rheology of the medium in which the particle is moving). The sizes of the particles, fins and flagella can range between 10's of nanometers to 1-10 millimeters in any dimension.

Examples of magnetic materials M1, M1', M2 that can be used include: iron, nickel, permalloy, cobalt, and others. For example, one may choose permalloy for the high permeability material and nickel for the lower permeability material, to ensure the flagella are not activated by the weak magnetic field B1 while the main-body/fins are affected by this field.

Approximated relationship between B1, B2, magnetic moments of materials M1, M1', M2 is provided, according to some embodiments of the invention. The relationship between B1, B2 and the permeabilities of materials M1, M1', M2 can be approximately described as follows.

Assuming that B1 has to be high enough to a generate torque t1 strong enough to cause particle [600] rotation, i.e. greater than a certain threshold (t1>T1) dependent on the rheology of surrounding medium and on particle size and shape.

t1 is approximated as:

$$t1 = C * B1 * Mt, \qquad Eq.\ 2$$

where

- Mt is the total magnetic moment of the particle; Mt can be approximated as a linear combination of M1 (magnetic moment of the main-body) and M1' (magnetic moment of the fins); this is the effective magnetic moment of the main-body and fins;
- the scale factor C depends on the angle between the external field and the particle axis (among other factors).

In order to trigger a rotation:

$$C*B1*Mt > T1 \qquad Eq.\ 3$$

Assuming B1+B2 are required to generate a torque t2 on the elastomer flagella, which is large enough to trigger a change in flagella shape. t2 is approximated as:

$$t2 = A*(B1+B2)*M2 \qquad Eq.\ 4$$

- the scale factor A is dependent on the flagella shape and angle in relation to the external magnetic field, among other factors.

In order to trigger a change in flagella shape:

$$A*(B1+B2)*M2 > T2 \qquad Eq.\ 5$$

where T2 is the threshold torque required for shape change, dependent on elastomer properties and environment rheology.
Let's denote:

$$B2=B1*N \qquad Eq.\ 6$$

- where, N is a scale factor.

So,

$$A*(N+1)*B1*M2 > T2 \qquad Eq.\ 7$$

However, it is also required that:

$$A*(B1)*M2 < T2 \qquad Eq.\ 8$$

i.e., the flagella do not get activated without the large field component B2.
Let's assume that:

$$T1=T2*D \qquad Eq.\ 9$$

- where D is a scale factor.

Then by combining *Eq. 3* and *Eq. 8* one gets:

$$C*B1*Mt>T2*D > A*B1*M2*D \qquad Eq.\ 10$$

and therefore,

$$C/(AD) > M2/Mt \qquad Eq.\ 11$$

This means that as long as M2 is not too high relative to Mt, the flagella cannot be activated by the field B1 alone.
A practical example for these measures. Assuming C=1 (equivalent to embedding the scale factor C in Mt), A=1 (equivalent to embedding scale factor A in M2). T1, T2, D are a given (i.e., physical parameters imposed on us). Mt, M2, B1, B2 are parameters one can choose.

Assuming one chooses a material generating total magnetic moment Mt for the particle main-body and fins. Denote Y=B1*Mt/T1. Since Mt, T1 have already been defined or chosen, one can now choose B1 = (T1*Y)/Mt, to satisfy Y > 1, so *Eq. 3* is satisfied.

By choosing N to be D+1, so B2=(D+1)*B1. If one can choose magnetic material M2 so that M2=Mt/(Y*D), where Y>1, then *Eq. 11* and *Eq. 8* are satisfied, substituting into *Eq. 5* so it is satisfied:

$$M2*(D+1)B1 = (Mt/(YD))*(D+1)B1 = Mt/(YD)*(D+1)*YT1/Mt$$

$$= (T1+ T1/D) > T2 = T1/D \qquad \qquad Eq.\ 12$$

In other terms, one needs to choose M2 and Mt so that M2/Mt scales inversely with D=T1/T2. Since M2 and Mt scale with the respective materials' magnetic permeability, M2 and Mt can be set to meet the above criteria by appropriate choice of materials.

If D (i.e. T1/T2) ranges between 1/100 and 100 (a wide range encompassing nearly all practical ratios in a physical scenario), one needs to choose a pair of materials M2, Mt whose permeability ratio scales inversely (between 100 and 1/100). Multiple examples of such materials exist with a wide range of permeability ratios (such as nickel vs. permalloy) to readily select suitable materials for a desired ratio. If D≥1 then one chooses Mt to be based on the higher permeability material, and M2 to be based on the lower permeability material. If D<1 then M2 is based on the higher permeability material, and Mt is based on the lower permeability material.

**EXAMPLE 2.**

According to some embodiments, a system is provided configured to release payloads (e.g., drug, therapeutic entities) encapsulated in a particle using an external magnetic signal, and based on a combination of elastomer-based membranes that are used to contain/release the payload.

The particle [200,300,400,500,550] as shown in **Figs. 2A-2B**, **3A-3B**, **4A-4B**, **5A-5B** and **5C** is comprised of:

- the main-body [210,310,410,510,560] of the particle, with a cavity [211,311,411,511,561] configured for containing the payload.
- a membrane [220,320,420,520,562] made of an elastomer with embedded MNPs;

the membrane can be attached to the cavity bottom in a spring-like fashion [220] (as illustrated in **Figs. 2A-2B**), configured to push a tray [214] on which the payload is accommodated; the membrane can be used to as a lid [320] configured to seal the cavity and prevent free payload diffusion (as illustrated in **Figs. 3A-3B** and **5C**); or the membrane can be designed as an arm [420,520] (as illustrated in **Figs. 4A-4B** and **5A-5B**), configured to open and close a sealing lid [412,512] of the cavity.

According to some embodiments, when there is no external magnetic field the membrane is in its default relaxed position, preventing (or at least not facilitating) payload diffusion out of the particle (meaning out of the cavity).

According to some embodiments, when a specific external magnetic field is applied, the membrane either:

- pushes the payload out of the cavity (as in **Fig. 2B**),
- folds to open the cavity and allow diffusion (as in **Fig. 3B** and **5C**), or
- pushes/pulls the sealing lid to open cavity (as in **Figs. 4B** and **5B**).

According to some embodiments, the several setups can be combined; i.e., two membranes - one opening/closing the cavity and the other pushing the payload out.

According to some embodiments, the device can be used in combination with magnetic particles (carrying the payload), which are propelled in viscoelastic media using an external rotating electromagnetic field. In this case, the entire particle is configured to rotate around its axis under the influence of the external rotating magnetic field. The plane of field rotation is orthogonal to the direction of motion. This rotation propels the particle forward like a corkscrew. According to some embodiments, inverting the direction of rotation of magnetic field propels the particle backwards, respectively.

The challenge is to ensure such a rotating external magnetic field does not activate the payload release mechanism described above. The solution: the particle body contains magnetic material M1. In contrast, the material of the elastomer membranes involved in the payload release mechanism comprise the embedded MNPs of magnetic material M2.

The external magnetic field has two components:

$$B=B1+B2 \qquad Eq.\ 13$$

- where B1 is the steering and propulsion rotating magnetic field component.

The goal is to prevent this component from activating the drug release elastomer membranes when B2=0.

Three methods are provided to prevent this, which can be applied individually or in combination:

The exact direction of the field B2, required to activate the elastomer membranes, can be accurately designed (as part of the elastomer membrane design and its position on the particle [550]). In an example of an elastomer [562] design as in **Fig. 5C,** where the planar elastomer membrane only changes its configuration when the vector of external magnetic field is not parallel to the two-dimensional plane of the membrane [562]. Accordingly, when the membrane [562] is positioned on the particle [550] so that it is orthogonal to the particle axis of rotation (i.e., parallel to the plane of the external rotating magnetic field), then as long as there is no sizeable vector component of B in the direction of main-body [560] motion, the elastomer membranes are not activated, and the payload is not released.

According to some embodiments, one can design the particle (i.e., choose the materials M1, M2) so it is capable of propulsion by B1 of low amplitude. In this design, the magnetic elastomer is not activated under field B1 due to the magnetic moment of material M2, which is low compared to the minimal torque required for elastomer activation, while the particle main-body keeps rotating with the field B1, due to the magnetic moment of material M1 (which is high enough compared to the minimal torque required for particle rotation). Only when B2 >>0 and B is substantially greater than B1, the magnetic elastomer in the membranes is activated and triggers payload release on demand. This can be done by appropriate choice of materials M1, M2 and fields B1, B2, as described in Example 1 above.

According to some embodiments, when magnetic field B1 rotates within a predefined operational plane and/or volume, which may be located inside a patient body, at a certain frequency F1, material M2 can be chosen by design such that, it responds to changes in an external magnetic field more slowly than the frequency F1 of the rotating field B1 (i.e., greater magnetic viscosity).

This choice can be combined with a specific membrane design that requires more time to change its shape in response to the change in external magnetic field. For example, when properly positioned in reference to the elastomer membrane (e.g., orthogonally to the membrane plane), the external field may exert aggregate torque t1 on the elastomer membrane (net of internal resistive forces in response to the shape deformation, which depend on the dynamic moduli of the elastomer membrane). The membrane starts deforming from a stationary position. It takes a minimal time x for the membrane to reach its fully extended position, which will allow payload diffusion. However, if the rotation frequency of the external field is high enough, then within time <<x the external magnetic field has rotated to a new angle relative to the membrane, at which the field no longer activates the membrane as the field component orthogonal to the membrane plane is lower than the threshold torque necessary for membrane activation. Therefore, the membrane never reaches its fully activated state. That means that as long as the external field is rotating, the elastomer never "catches up" with it, so it is not activated, and the payload is not released. A long fixed pulse B2 is activated only at the desired moment of payload release. This pulse is long enough to cross the threshold of the response time x for the elastomer membrane. Therefore, the elastomer membrane is activated, and the payload is released on demand.

According to some embodiments, all three of the above options can be combined by using a rectangular, double exponential, damped sinewave pulse or a combination thereof, within a range of 10 millisecond to 1 minute pulse of a high magnetic field in a direction orthogonal to the plane of the rotating low magnetic field.

The strength of the relevant magnetic fields B1, B2 can range anywhere between single-digit Gauss to single-digit Tesla (depending on particle size and geometry, materials used, rheology of medium in which particle is moving).

The size of the particles can range between 10's of nanometers to 10's of mm's in any dimension.

Examples of magnetic materials M1, M2 defined above that can be used include iron, nickel, permalloy, cobalt, and others. For example, one may choose permalloy for higher permeability and nickel for lower permeability to make sure the membrane is not activated by the weak magnetic field B1, while the body is affected by this field.

## Examples for manufacturing thin elastomer layers

The following manufacturing methods for elastomer-based membranes and magnetic particles do not form part of the claimed invention.

The motility appendages described above (various flagella as in Example 1) as well as the payload release control membranes/springs described above (as in Example 2) include, but are not limited to, magnetic polymer composites comprising a base polymer and a dispersed magnetic phase.

For example, flagella for the device can be manufactured via a template-based or template-free magnetic assembly. Specifically, the 'frog legs', accordion, or 'fin'-shaped flagella can be manufactured using casting and/or molding techniques.

In a representative procedure, a preformed mold and/or cast is filled with a solution or neat liquefied polymer of choice (ex., polydimethylsiloxane) followed by addition of magnetic micro/nanoparticles to create a suspension.

The resulting suspension is allowed to cure in the presence of an external magnetic field or alternative source of energy (ex., ultrasound) in order to ascertain unified and/or patterned particle distribution throughout the polymer to furnish in the targeted magnetoactive elastomer material.

The resulting flagella can have 'shape-memory' features ("Stimulus responsive shape-memory materials: A review," Materials and Design 33 (2012), pages 577-640) and be capable of being propelled by external magnetic field(s) as exemplified in **Figs. 2A, 2B,** 2C. Similarly, the 'shape-memory' and topology features of the elastomer-based membrane or of the elastomer-based spring can be achieved using the same manufacturing techniques. Stimulus-responsive shape-memory materials respond to a particular stimulus, such as heat, chemical, magnetic, electric, mechanical and light. The response may be reversible. While in most stimulus-responsive materials, the result is limited to a change in certain physical/chemical properties, stimulus-responsive shape memory materials (SMMs) recover their original shape, after being quasi-plastically distorted. SMMs are ideal for integrated systems, where the materials are actuated and generate a reactive motion. SMMs, include for example shape memory alloys (SMAs) and shape memory polymers (SMPs). SMMs also include ceramics, gels and combinations of these materials. Shape-memory materials and the stimulus to which they respond are included in embodiments of this invention.

The solid particle body can range in size from a few nanometers to a few micrometers and exhibit specific and tunable magnetic properties. The adjustable magnetic features are diamagnetic, paramagnetic, superparamagnetic and ferromagnetic, depending on chemical composition, crystalline structure and size of the particles used. More specifically, representative examples of particle candidates include neodymium (ex., $Nd_2Fe_{14}B$ ("A magnetic membrane actuator in composite technology utilizing diamagnetic levitation," IEEE Sens. J. 13 (2013), pages 2786-2797), carbon-coated Fe ("Microfabrication of magnetically actuated PDMS-fron composite membranes," Microelectr. Engineer. 98 (2012), pages 607-609), iron (II/III) oxides ("Magnetically-actuated artificial cilia for microfluidics propulsion," Lab Chip. 11 (2011), pages 2002-2010), cobalt alloy(s) ("A facile template-free approach to magnetodriven multifunctional artificial cilia," Appl. Mater. Interfaces 2 (2010), pages 2226-2230), etc.)

These particles are incorporated into a compatible polymer matrix, such as polydimethylsiloxane (PDMS) ("Magnetically actuated micropumps using an Fe-PDMS composite membrane," Proc. SPIE Conf. Smart. Struc. Mater. 2006, p. 617213). Additional examples of elastic polymer matrices include but are not limited to poly n-butylacrylate (PnBA) ("Magnetically-actuated artificial cilia for microfluidics propulsion," Lab Chip. 11 (2011), pages 2002-2010), poly(styrene-block-isoprene-block-styrene) ("A facile template-free approach to magnetodriven multifunctional artificial cilia," Appl. Mater. Interfaces 2 (2010)), and SU-8 (a commonly used epoxy-based negative photoresist polymer) ("Single cell manipulation using ferromagnetic composite microtransporters," Appl. Phys. Lett. 96 (2010), 043705).

Specific manufacturing technologies to incorporate particles of interest into magnetoactive elastomers include but are not limited to:

- casting as a standalone process or using sacrificial coating (e.g., polyethyleneglycol (PEG); polyvinylacrylate (PVA); or polycarbonate);
- molding/casing to produce a pre-determined shape with embedded particles followed by laser-, chemical- or other etching techniques to achieve the desired topology;
- photopatterning,
- self-assembly under magnetic field, and iv) lithography ("A review of magnetic composite polymers applied to microfluidics devices," J. Electrochem. Soc. 161 (2014), pages B3173-B3183).

## Claims

1. A device (100, 200, 300, 400, 500, 550, 600) for implanting in a biological tissue and adapted to move in a viscoelastic media, the device comprising:

   a main-body (110, 210, 310, 410, 510, 560, 610) comprising a first material and having a direction in the viscoelastic media, and being configured to change its direction in the viscoelastic media upon application thereto of a first stimulus field at a first threshold; and
   one or more memory shaped elements (120, 220, 320, 420, 520, 562, 620) externally attached to the main-body, each of said memory shaped elements having a first configuration and comprising a second material being a stimulus-responsive shape-memory material, said second material comprises an elastomer, and wherein the memory shaped element is configured to adopt a second configuration upon application thereto of a second stimulus field at a second threshold, thereby propelling the main-body through the viscoelastic media.

2. The device (100, 200, 300, 400, 500, 550, 600) of claim 1, wherein the second material is different from the first material.

3. The device (100, 200, 300, 400, 500, 550, 600) of claim 1, wherein the first and second stimulus fields are of the same nature and the same direction; and wherein the second threshold is larger than the first threshold.

4. The device (100, 200, 300, 400, 500, 550, 600) of claim 1, wherein the material of at least some of the memory shaped elements (120, 220, 320, 420, 520, 562, 620) are different one from another.

5. The device (100, 200, 300, 400, 500, 550, 600) of claim 1, wherein at least one of first and second materials comprises a form of micro- or nano- particles.

6. The device (100, 200, 300, 400, 500, 550, 600) of claim 1, the first or second configuration of the memory shaped element (120, 220, 320, 420, 520, 562, 620) is selected from a group consisting of: an elongated shape, a film, a wire, a string, a strip, a plug, a sheet, a membrane, flagellum, coil, helix, arm, joint and any combination thereof.

7. The device (100, 200, 300, 400, 500, 550, 600) of claim 1, wherein the application of the second stimulus field comprises cycles of the second stimulus field above and below the second threshold.

8. The device (600) of claim 1, wherein the main-body (610) further comprises at least two fins (630), configured to steer the direction of the main-body.

9. The device (600) of claim 8, wherein the fins (630) comprise:

   a) the first material, and are optionally:
   b) externally and symmetrically attached to the main-body (610); or
   c) configured to tilt relative to the main-body (610).

10. The device (600) of claim 9, wherein the fins (630) comprise a polarity direction at an angle relative to the main-body (610).

11. The device (200, 300, 400, 500, 550) of claim 1, wherein the main-body (210, 310, 410, 510, 560) further comprises a sealable cavity (211, 311, 411, 511, 561) and when the memory shaped element (220, 320, 420, 520, 562) is in the first configuration the cavity is closed and in the second configuration the cavity is open.

12. The device (200, 300, 400, 500, 550) of claim 11, wherein optionally:

   a) the sealable cavity (211, 311, 411, 511, 561) is configured to temporarily accommodate at least one of: a therapeutic entity, a therapeutic load, a diagnostic load, or a combination thereof; or
   b) the sealable cavity is configured to temporarily accommodate an explosion material, configured to propel the main-body; or
   c) further comprising a sensitive sealing lid (320, 562), configured to temporarily seal the cavity; wherein the sensitive sealing lid (320, 562) is configured to be opened responsive to an environmental threshold other than one of said stimulus fields; or
   d) the memory shaped element is configured as a sealing lid (320, 562) for the cavity; and wherein configuration of the MSE opens and/or closes the sealable cavity; or
   e) the memory shaped element constitutes a first arm (420, 520) configured to pull and/or push a sealing lid (412, 512) of the cavity upon application of the second stimulus field.

13. The device (400, 500) of claim 12, wherein the memory shaped element constitutes a first arm (420, 520) configured to pull and/or push a sealing lid (412, 512) of the cavity upon application of the second stimulus field, wherein the first arm comprises at least one element selected from: a spring, a helical spring, a leaf spring, a rod, a shaft, a pole and a bar.

14. The device (200) of claim 1, wherein the main-body (210) further comprises a cavity (211) and wherein the memory shaped element constitutes comprises an arm (220), configured to push a substance accommodated within the cavity out of the cavity upon application of the second stimulus field.

15. A system (700) comprising:

the device (710) of any one of claims 1 to 14; and
a remote controlling module (720) configured to control the application of the first stimulus field and the second stimulus field.

**Patentansprüche**

1. Vorrichtung (100, 200, 300, 400, 500, 550, 600) zum Implantieren in ein biologisches Gewebe, das dazu adaptiert ist, sich in viskoelastischen Medien zu bewegen, wobei die Vorrichtung wie folgt umfasst:

einen Hauptkörper (110, 210, 310, 410, 510, 560, 610), der ein erstes Material umfasst und in den viskoelastischen Medien eine Richtung hat und dazu konfiguriert ist, seine Richtung in den viskoelastischen Medien zu ändern, wenn bei einem ersten Schwellenwert ein erstes Reizfeld hieran angelegt wird; und
ein oder mehrere Formgedächtnis-Elemente (120, 220, 320, 420, 520, 562, 620), die jeweils von außen an dem Hauptkörper befestigt sind, wobei jedes der Formgedächtnis-Elemente eine erste Konfiguration hat und ein zweites Material umfasst, das ein auf Reize reagierendes Formgedächtnis-Material ist, wobei das zweite Material einen Elastomer umfasst und wobei das Formgedächtnis-Element dazu konfiguriert ist, eine zweite Konfiguration anzunehmen, wenn bei einem zweiten Schwellenwert ein zweites Reizfeld hieran angelegt wird, wodurch der Hauptkörper durch die viskoelastischen Medien vorwärtsgetrieben wird.

2. Vorrichtung (100, 200, 300, 400, 500, 550, 600) nach Anspruch 1, wobei das zweite Material von dem ersten Material verschieden ist.

3. Vorrichtung (100, 200, 300, 400, 500, 550, 600) nach Anspruch 1, wobei das erste Reizfeld und das zweite Reizfeld wesens- und richtungsgleich sind; und wobei der zweite Schwellenwert größer als der erste Schwellenwert ist.

4. Vorrichtung (100, 200, 300, 400, 500, 550, 600) nach Anspruch 1, wobei das Material mindestens mancher der Formgedächtnis-Elemente (120, 220, 320, 420, 520, 562, 620) voneinander verschieden ist.

5. Vorrichtung (100, 200, 300, 400, 500, 550, 600) nach Anspruch 1, wobei mindestens eines von dem ersten und zweiten Material eine Form von Mikro- oder Nanopartikeln umfasst.

6. Vorrichtung (100, 200, 300, 400, 500, 550, 600) nach Anspruch 1, wobei die erste oder zweite Konfiguration des Formgedächtnis-Elements (120, 220, 320, 420, 520, 562, 620) aus der Gruppe bestehend aus Folgenden ausgewählt ist: einer länglichen Form, einer Folie, einem Draht, einem Faden, einem Streifen, einem Stopfen, einem Blatt, einer Membran, einer Geißel, einer Spule, einer Helix, eines Arms, eines Gelenks und Kombinationen davon.

7. Vorrichtung (100, 200, 300, 400, 500, 550, 600) nach Anspruch 1, wobei die Anlegung des zweiten Reizfeldes Zyklen des zweiten Reizfelds oberhalb und unterhalb des zweiten Schwellenwertes umfasst.

8. Vorrichtung (600) nach Anspruch 1, wobei der Hauptkörper (610) ferner mindestens zwei (630) Rippen umfasst, die dazu konfiguriert sind, den Hauptkörper in seine Richtung zu lenken.

9. Vorrichtung (600) nach Anspruch 8, wobei die Rippen (630) ferner wie folgt umfassen:

a) das erste Material; und wobei sie sind wahlweise
b) von außen und symmetrisch an dem Hauptkörper (610) befestigt sind; oder sie
c) dazu konfiguriert sind, in Bezug auf den Hauptkörper (610) abgekippt zu werden.

10. Vorrichtung (600) nach Anspruch 9, wobei die Rippen (630) in einem Winkel in Bezug auf den Hauptkörper (610) eine Polaritätsrichtung umfassen.

11. Vorrichtung (200, 300, 400, 500, 550) nach Anspruch 1, wobei der Hauptkörper (210, 310, 410, 510, 560) ferner einen verschließbaren Hohlraum (211, 311, 411, 511, 561) umfasst und wobei der Hohlraum geschlossen ist, wenn das Formgedächtnis-Element (220, 320, 420, 520, 562) in der ersten Konfiguration ist und geöffnet, wenn es in der zweiten Konfiguration ist.

12. Vorrichtung (200, 300, 400, 500, 550) nach Anspruch 11, wobei wahlweise Folgendes gilt:

a) der verschließbare Hohlraum (211, 311, 411, 511, 561) ist dazu konfiguriert, mindestens eines hiervon vorrübergehend hierin unterzubringen: eine therapeutische Entität, eine therapeutische Last, eine diagnostische Last oder eine Kombination davon; oder

b) der verschließbare Hohlraum ist dazu konfiguriert, ein Explosionsmaterial vorrübergehend hierin unterzubringen, das dazu konfiguriert ist, den Hauptkörper vorwärtszutreiben; oder

c) ferner umfassend einen empfindlichen Verschlussdeckel (320, 562), der dazu konfiguriert ist, den Hohlraum vorrübergehend zu verschließen; wobei der empfindliche Verschlussdeckel (320, 562) dazu konfiguriert ist, auf einen umweltbedingten Schwellenwert anstatt auf eines der Reizfelder reagierend geöffnet zu werden; oder

d) das Formgedächtnis-Element ist als ein Verschlussdeckel (320, 562) für den Hohlraum konfiguriert und wobei die Konfiguration des Formgedächtnis-Elements den verschließbaren Hohlraum öffnet und schließt; oder

e) das Formgedächtnis-Element bezeichnet einen ersten Arm (420, 520), der dazu konfiguriert ist, an einem Verschlussdeckel (412, 512) des Hohlraums zu ziehen und/oder dagegen zu drücken, wenn das zweite Reizfeld angelegt wird.

13. Vorrichtung (400, 500) nach Anspruch 12, wobei das Formgedächtnis-Element einen ersten Arm (420, 520) bezeichnet, der dazu konfiguriert ist, an einem Verschlussdeckel (412, 512) des Hohlraums zu ziehen und/oder dagegen zu drücken, wenn das zweite Reizfeld angelegt wird, wobei der Arm mindestens ein Element, das unter Folgenden ausgewählt ist, umfasst: eine Feder, eine Schraubenfeder, eine Blattfeder, einen Stab, einen Schaft und einen Balken.

14. Vorrichtung (200) nach Anspruch 1, wobei der Hauptkörper (210) ferner einen Hohlraum (211) umfasst und wobei das Formgedächtnis-Element einen Arm (220) darstellt/umfasst, der dazu konfiguriert ist, eine Substanz, die in dem Hohlraum untergebracht ist, aus dem Hohlraum herauszudrücken, wenn das zweite Reizfeld angelegt wird.

15. System (700) umfassend wie folgt:

Vorrichtung (710) nach einem der Ansprüche 1 bis 14; und
ein Fern-Steuermodul (720), das dazu konfiguriert ist, die Anlegung des ersten Reizfeldes und des zweiten Reizfeldes zu steuern.

## Revendications

1. Dispositif (100, 200, 300, 400, 500, 550, 600) d'implantation dans un tissu biologique, adapté pour se déplacer dans un milieu viscoélastique, le dispositif comprenant :

un corps principal (110, 210, 310, 410, 510, 560, 610) comprenant un premier matériau et ayant une direction dans le milieu viscoélastique, et étant configuré pour changer de direction dans le milieu viscoélastique lorsqu'un premier champ de stimulus à un premier seuil lui est appliqué ; et
un ou plusieurs éléments à mémoire de forme (120, 220, 320, 420, 520, 562, 620) fixés à l'extérieur du corps principal, chacun desdits éléments à mémoire de forme ayant une première configuration et comprenant un second matériau ayant une mémoire de forme et étant sensible au stimulus, ledit second matériau comprenant un élastomère, et l'élément à mémoire de forme étant configuré pour adopter une seconde configuration lors de l'application sur celui-ci d'un second champ de stimulus à un second seuil, propulsant ainsi le corps principal à travers le milieu viscoélastique.

2. Dispositif (100, 200, 300, 400, 500, 550, 600) selon la revendication 1, dans lequel le second matériau est différent du premier matériau.

3. Dispositif (100, 200, 300, 400, 500, 550, 600) selon la revendication 1, dans lequel les premier et second champs de stimulus sont de même nature et de même direction ; et dans lequel le second seuil est supérieur au premier seuil.

4. Dispositif (100, 200, 300, 400, 500, 550, 600) selon la revendication 1, dans lequel le matériau d'au moins certains des éléments à mémoire de forme (120, 220, 320, 420, 520, 562, 620) sont différents les uns des autres.

5. Dispositif (100, 200, 300, 400, 500, 550, 600) selon la revendication 1, dans lequel au moins l'un des premier et second matériaux comprend une forme de micro- ou nanoparticules.

6. Dispositif (100, 200, 300, 400, 500, 550, 600) selon la revendication 1, dans lequel la première ou la seconde configuration de l'élément à mémoire de forme (120, 220, 320, 420, 520, 562, 620) est sélectionnée dans un groupe constitué de : une forme allongée, un film, un fil, une ficelle, une bande, un bouchon, une feuille, une membrane, un flagelle, une bobine, une hélice, un bras, une articulation et toute combinaison de ceux-ci.

7. Dispositif (100, 200, 300, 400, 500, 550, 600) selon la revendication 1, dans lequel l'application du second champ de stimulus comprend des cycles du second champ de stimulus au-dessus et au-dessous du second seuil.

8. Dispositif (600) selon la revendication 1, dans lequel le corps principal (610) comprend en outre au moins deux ailettes (630), configurées pour orienter la direction du corps principal.

9. Dispositif (600) selon la revendication 8, dans lequel les ailettes (630) comprennent :

    a) le premier matériau, et sont éventuellement :
    b) fixées à l'extérieur et symétriquement au corps principal (610) ; ou
    c) configurées pour s'incliner par rapport au corps principal (610).

10. Dispositif (600) selon la revendication 9, dans lequel les ailettes (630) comprennent une direction de polarité formant un angle par rapport au corps principal (610).

11. Dispositif (200, 300, 400, 500, 550) selon la revendication 1, dans lequel le corps principal (210, 310, 410, 510, 560) comprend en outre une cavité pouvant être rendue étanche (211, 311, 411, 511, 561) et lorsque l'élément à mémoire de forme (220, 320, 420, 520, 562) est dans la première configuration, la cavité est fermée et dans la seconde configuration, la cavité est ouverte.

12. Dispositif (200, 300, 400, 500, 550) selon la revendication 11, dans lequel optionnellement :

    a) la cavité pouvant être rendue étanche (211, 311, 411, 511, 561) est configurée pour accueillir temporairement au moins l'un parmi : une entité thérapeutique, une charge thérapeutique, une charge de diagnostic, ou une combinaison de celles-ci ; ou
    b) la cavité pouvant être rendue étanche est configurée pour recevoir temporairement un matériau explosif, configuré pour propulser le corps principal ; ou
    c) comprenant en outre un couvercle d'étanchéité sensible (320, 562), configuré pour rendre la cavité temporairement étanche ; dans lequel le couvercle d'étanchéité sensible (320, 562) est configuré pour être ouvert en réponse à un seuil environnemental autre que l'un desdits champs de stimulus ; ou
    d) l'élément à mémoire de forme est configuré comme un couvercle d'étanchéité (320, 562) pour la cavité ; et dans lequel la configuration de l'élément à mémoire de forme ouvre et/ou ferme la cavité pouvant être rendue étanche ; ou
    e) l'élément à mémoire de forme constitue un premier bras (420, 520) configuré pour tirer et/ou pousser un couvercle d'étanchéité (412, 512) de la cavité lors de l'application du second champ de stimulus.

13. Dispositif (400, 500) selon la revendication 12, dans lequel l'élément à mémoire de forme constitue un premier bras (420, 520) configuré pour tirer et/ou pousser un couvercle d'étanchéité (412, 512) de la cavité lors de l'application du second champ de stimulus, dans lequel le premier bras comprend au moins un élément choisi parmi : un ressort, un ressort hélicoïdal, un ressort à lames, une tige, un arbre, un poteau et une barre.

14. Dispositif (200) selon la revendication 1, dans lequel le corps principal (210) comprend en outre une cavité (211) et dans lequel l'élément à mémoire de forme comprend un bras (220), configuré pour pousser une substance logée à l'intérieur de la cavité vers l'extérieur de la cavité lors de l'application du second champ de stimulus.

15. Système (700) comprenant :

le dispositif (710) selon l'une quelconque des revendications 1 à 14 ; et
un module de commande à distance (720) configuré pour contrôler l'application du premier champ de stimulus et du second champ de stimulus.

100

110

120

**Fig. 1A**

100

110

120

SF

**Fig. 1B**

100

110

120

Under SF

**Fig. 1C**

**Fig. 2A**

**Fig. 2B**

**Fig. 3A**

**Fig. 3B**

400

410

412

420

411

**Fig. 4A**

400

412

420

410

411

SF

**Fig. 4B**

500

512

520

510

511

**Fig. 5A**

500

512

520

510

511

SF

**Fig. 5B**

550

561

560

562

SF

**Fig. 5C**

600

610

630

N

N

S

N

N

S

620

Fig. 6A

600

610

N

N

S

N

S

630

B

SF

Fig. 6B

600

610

N

S

N

S

SF

630

Fig. 6C

Fig. 6F

Fig. 6E

Fig. 6D

**Fig. 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8768501 B
- US 20080255543 A
- US 20110200434 A
- US 20070225634 A

### Non-patent literature cited in the description

- **MISHRA et al.** Selective and directional actuation of elastomer films using chained magnetic nanoparticles. *Nanoscale,* 2016, vol. 8, 1309-1313
- Stimulus responsive shape-memory materials: A review. *Materials and Design,* 2012, vol. 33, 577-640
- A magnetic membrane actuator in composite technology utilizing diamagnetic levitation. *IEEE Sens. J,* 2013, vol. 13, 2786-2797
- Microfabrication of magnetically actuated PDMS-fron composite membranes. *Microelectr. Engineer.,* 2012, vol. 98, 607-609
- Magnetically-actuated artificial cilia for microfluidics propulsion. *Lab Chip.,* 2011, vol. 11, 2002-2010
- A facile template-free approach to magnetodriven multifunctional artificial cilia. *Appl. Mater. Interfaces,* 2010, vol. 2, 2226-2230
- Magnetically actuated micropumps using an Fe-PDMS composite membrane. *Proc. SPIE Conf. Smart. Struc. Mater.,* 2006, 617213
- A facile template-free approach to magnetodriven multifunctional artificial cilia. *Appl. Mater. Interfaces,* 2010, vol. 2
- Single cell manipulation using ferromagnetic composite microtransporters. *Appl. Phys. Lett.,* 2010, vol. 96, 043705
- A review of magnetic composite polymers applied to microfluidics devices. *J. Electrochem. Soc.,* 2014, vol. 161, B3173-B3183